# FASCICULE DE BREVET EUROPEEN

(11) **EP 1 695 691 B1**
(45) Date de publication et mention de la délivrance du brevet: **30.09.2009**
(21) Numéro de dépôt: 05022601.8
(22) Date de dépôt: 17.06.1999
(51) Int. Cl.: A61K 8/34, A61K 8/37, A61K 8/41, A61K 8/42, A61K 8/44, A61K 8/46, A61K 8/63, A61K 8/73, A61K 8/81, A61K 8/92, A61Q 5/02

(54) **Composition moussante pour le lavage et le traitement des cheveux**
Schäumende Zusammensetzung zur Haarreinigung und Haarbehandlung
Foaming composition for hair cleaning and hair treatment

(30) Priorité: 19.06.1998 FR 9807802
(43) Date de publication de la demande: 30.08.2006
(62) Demande divisionnaire de: 99925117.6
(73) Titulaire: Galderma S.A., 6330 Cham (CH)
(72) Inventeur: Preuilh, Isabelle, 06110 Le Cannet (FR); Guise, Anne-Emmanuelle Les Jardins de la Prefect., 76000 Rouen (FR); Willcox, Nathalie, 06650 Le Rouret (FR)
(74) Mandataire: Casalonga, Axel

(56) Documents cités:
- WO-A-96/27376
- US-A- 5 352 437
- US-A- 5 661 118
- SCHRADER K: "Grundlagen und Rezepturen der Kosmetika" GRUNDLAGEN UND REZEPTUREN DER KOSMETIKA, XX, XX, 1989, pages 680-709, XP002286009

## Description

La présente invention est relative à de nouvelles compositions moussantes de lavage et traitement des cheveux et/ou du cuir chevelu, contenant du propionate de clobétasol comme principe actif, des tensioactifs anioniques et amphotères particuliers et de l'éthanol, ainsi qu'au procédé de traitement mettant en oeuvre de telles compositions.

Parmi toutes les maladies chroniques de la peau, le psoriasis est une des affections cutanées les plus communes. Cette maladie s'observe chez 1,4 à 2,9% de la population. Le cuir chevelu est l'un des sièges de prédilection du psoriasis; ce dernier provoque essentiellement des érythèmes, des desquamations, des hyperkératoses, des prurits et peut également être responsable d'une réduction de la densité capillaire. Les traitements utilisés jusqu'à présent font appel à l'acide salicylique, aux stéroïdes locaux, à l'anthraline ou au goudron de houille ou de bois. Ces traitements sont déplaisants, notamment lors de l'application de goudron, et nécessitent de longues applications, notamment lorsque des pommades capillaires sont utilisées.

En vue d'améliorer la qualité de vie du patient, sans toutefois diminuer l'effet thérapeutique du traitement, des compositions moussantes contenant des corticostéroïdes ont été mises au point. Plus particulièrement, le temps d'application de ces compositions moussantes est réduit par rapport au traitement classique.

Le brevet BE 84515 décrit une composition contenant de l'hydrocortisone dans un mélange solvant constitué de 15 à 60% d'alcool aliphatique, 15 à 60% de propylèneglycol et de 5 à 60% d'un tiers agent solubilisant pris dans le groupe constitué par le salicylate d'hydroxy-2 éthyle, le dipropylcétone et le chlorure de diméthyl cocobenzylammonium.

Le brevet EP 0 325 949 décrit une solution comprenant au moins 2,5% de corticostéroïdes, de 25 à 80% d'un tensioactif non ionique, de 0 à 70% d'éthanol, de 0 à 70% de propylèneglycol, et un agent antimicrobien.

Néanmoins, les solvants présents dans ces compositions ont tendance à s'évaporer très rapidement. De plus, la fluidité de ces compositions rend l'application difficile et il est souvent nécessaire d'appliquer ces compositions par frictionnement pour permettre une pénétration efficace des principes actifs, ce qui a pour conséquence d'irriter encore d'avantage l'épiderme, ou d'appliquer ces compositions et de les laisser agir pendant plusieurs heures, ce qui entraîne des désagréments pour le patient.

Pour éviter l'évaporation du solvant, il a été proposé dans le brevet WO 9627376 une mousse contenant un corticostéroïde, un agent casseur de mousse, un propulseur et tampon. L'agent casseur de mousse est composé d'un alcool aliphatique, d'eau, d'un alcool gras et d'un tensioactif non ionique. Néanmoins, lorsque ces mousses sont appliquées sur des fibres kératiniques, leur aspect esthétique résultant n'est pas satisfaisant et le cuir chevelu situé sous les fibres kératiniques peut être insuffisamment traité.

La demanderesse a recherché des compositions ne présentant pas les inconvénients susmentionnés, c'est à dire une composition permettant d'améliorer la pénétration du principe actif, tout en étant d'une utilisation particulièrement aisée, présentant de bonnes propriétés cosmétiques.

US-A-5 661 118 décrit une composition de lavage des cheveux, contenant:
- un milieu aqueux (voir colonne 14, ligne 2)
- au moins un tensioactif anionique (voir revendication 1),
- au moins un tensioactif amphotère (voir revendication 1).

Cette base lavante est utilisée en combinaison avec un agent dermatologique, une (glyco)céramide (voir revendication 1). Il s'agit donc d'un shampooing thérapeutique.

Le pH est de 3-8 (voir colonne 13, lignes 8-9).

La demanderesse a maintenant mis au point une composition moussante présentant les propriétés précédemment mentionnées.

Les compositions de la présente invention, tout en permettant une bonne pénétration des principes actifs, présentent également, une amélioration des performances cosmétiques des compositions pour le traitement des affections cutanées ou des cheveux, les compositions de l'invention laissant les cheveux doux, souples et ne les rendant pas gras. De plus, ces compositions sont faciles à rincer.

La demanderesse a constaté également que les compositions moussantes de la présente invention permettaient d'obtenir, de façon surprenante, une mousse présentant des caractéristiques de volume et de compacité améliorées par rapport aux compositions moussantes pour le traitement des affections cutanées ou du cuir chevelu de l'art antérieur.

Il a également été constaté que, de façon surprenante, les compositions objets de la présente invention sont stables dans le temps, en permettant d'éviter la précipitation des principes actifs, et restent donc limpides.

L'un des objets de l'invention est donc constitué par une composition moussante pour le lavage et le traitement des cheveux et/ou du cuir chevelu.

Un autre objet de l'invention est un procédé de lavage et de traitement des cheveux et/ou du cuir chevelu mettant en oeuvre une telle composition.

Un objet de l'invention est également une composition selon l'invention pour son application comme médicament.

Un objet de l'invention est aussi l'utilisation des compositions de l'invention comme médicament et pour la fabrication d'un médicament destiné à traiter les affections cutanées ou des cheveux.

D'autres objets de l'invention apparaîtront à la lecture de la description et des exemples qui suivent.

La composition moussante de pH 5.5-6.5 pour le lavage et le traitement des cheveux et/ou du cuir chevelu est essentiellement caractérisée par le fait qu'elle contient dans un milieu aqueux:
- du propionate de clobétasol
- au moins un tensioactif anionique choisi parmi le laurylethersulfate de sodium et le laurylsulfate de sodium,
- au moins un tensioactif amphotère choisi parmi les cocobétaïnes, et
- l'éthanol.

Le principe actif particulièrement préféré parmi les corticoïdes est le 17-propionate de clobetasol.

Le principe actif peut être utilisé dans des proportions de 0,001 à 5%, préférentiellement entre 0,01 à 0,3% et plus préférentiellement entre 0,05 et 0,1% en poids par rapport au poids total de la composition.

Comme tensioactifs anioniques, on utilise selon l'invention les sels de sodium de laurylsulfates ou de lauryléthersulfates ou leurs mélanges.

Plus particulièrement, on préfère utiliser le lauryléthersulfate de sodium (2 moles OE), notamment celui commercialisé sous la dénomination "Texapon N70®", le lauryléthersulfate de sodium, particulièrement celui commercialisé sous la dénomination "Sipon AOS 225 UP®", et le laurylsulfate de sodium, notamment celui commercialisé sous la dénomination "Texapon K 12®".

Ces tensioactifs anioniques peuvent être utilisés dans des proportions comprises entre 0,05 et 50%, de préférence entre 1 et 30% et plus préférentiellement entre 2 et 25% de Matière Active (M.A.) en poids par rapport au poids total de la composition.

Comme tensioactifs amphotères, on utilise les cocobétaïnes et plus particulièrement la cocamidopropylbétaïne, notamment celle commercialisée sous la dénomination "Tegobétaïne F50®", la cocamidopropylhydroxysultaïne, notamment celle commercialisée sous la dénomination "Amonyl 675 SB®", et les cocoylbétaïnes, notamment celles commercialisées sous les dénominations "Dehyton AB 30®" et "Chimexane HC®".

Ces tensioactifs amphotères peuvent être utilisés dans des proportions comprises entre 0,01 et 30%, de préférence entre 0,5 et 20% et plus préférentiellement entre 1 et 15% en M.A. en poids par rapport au poids total de la composition.

Le ratio entre les proportions en M.A. des tensioactifs anioniques et des tensioactifs amphotères est préférentiellement compris entre 1 et 20, et plus préférentiellement entre 2 et 10.

L'agent propénétrant qui permet de faciliter la pénétration des principes actifs, est de préférence solubilisant de l'actif présent dans la composition selon l'invention.

L'agent propénétrant dans le cadre de la présente invention est l'éthanol.

Les agents propénétrants peuvent être utilisés dans des concentrations comprises entre 0,1 et 25% et préférentiellement comprises entre 5 et 10% en poids par rapport au poids total de la composition.

Le ratio entre la proportion en M.A. des tensioactifs anioniques et la proportion des agents propénétrants est préférentiellement compris entre 0,1 et 10, et plus préférentiellement entre 0,5 et 5, et encore plus préférentiellement entre 1 et 2.

Les compositions objets de la présente invention pourront être épaissies et leurs propriétés cosmétiques améliorées en y ajoutant par exemple des polymères cationiques, des polymères acryliques, des dérivés cellulosiques quaternisés ou non.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques et/ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire en nombre comprise entre 500 et 5.10⁶ environ, et de préférence comprise entre 10³ et 3.10⁶ environ.

Parmi les polymères cationiques, on peut citer plus particulièrement les protéines (ou hydrolysats de protéines) quaternisées et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment:
- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate";
- les hydrolysats de collagène portant des groupements chlorure de triméthylammonium et de triméthylstéarylammonium, vendus sous la dénomination de "Quat-Pro S" par la Société Maybroook et dénommés dans le dictionnaire CTFA"Steartrimonium Hydrolyzed Collagen";
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein";
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres:
- le "Croquat L" dont les groupements ammonium quaternaires comportent un groupement alkyle en C12;
- le "Croquat M" dont les groupements ammonium quaternaires comportent des groupements alkyle en C10-C18;
- le "Croquat S" dont les groupements ammonium quaternaires comportent un groupement alkyle en C 18;
- le "Crotein Q" dont les groupements ammonium quaternaires comportent au moins un groupe alkyle ayant de 1 à 18 atomes de carbone.

Ces différents produits sont vendus par la Société Croda.

D'autre protéines ou hydrolysats quaternisés sont par exemple ceux répondant à la formule: 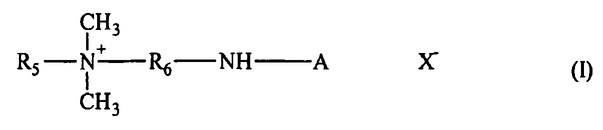 dans laquelle X^{⊖} est un anion d'un acide organique ou minéral, A désigne un reste de protéine dérivé d'hydrolysats de protéine de collagène, R₅ désigne un groupement lipophile comportant jusqu'à 30 atomes de carbone, R₆ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagent Hydrolysate" .

On peut encore citer les protéines végétales quaternisées telles que les protéines de blé, de maïs ou de soja: comme protéines de blé quaternisées, on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer:
(1) Les copolymères vinylpyrrolidone-acrylate a-méthacrylate de dialkylaminoalkyle quaternisés ou non, tels que les produits vendus sous la dénomination "Gafquat" par la Société ISP, comme par exemple Gafquat 734, 755 ou HS100 ou bien le produit dénommé "Copolymère 937". Ces polymères sont décrits en détail dans les brevets français 2 077 143 et 2 393 573.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyldiallylammonium.
   Les produits commercialisés répondant à cette définition sont plus particulierement les produits vendus sous la dénomination "Celquat SC 240", "Celquat L 200" et "Celquat H 100" par la Société National Starch.
(4) Les polysaccharides cationiques décrits plus particulièrement dans les brevets US 3 589 578 et 4 031 307 tel que les gommes de guar contenant des groupements cationiques trialkylammonium. On utilise par exemple des gommes de guar modifiées par un sel (par ex. chlorure) de 2,3-époxypropyl triméthylammonium.
   De tels produits sont commercialisés notamment sous les dénominations commerciales de JAGUAR C13 S, JAGUAR C 15, JAGUAR C 17 ou JAGUAR C162 par la société MEYHALL.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bishalohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé; agent réticulant étant utilisé dans des proportions allant de 0,025 à 0,35 mole par groupement amine du polyaminoamide; ces polyaminoamides peuvent être alcoylés ou, s'ils comportent une ou plusieurs fonctions amines tertiaires, quaternisées. De tels polymères sont notamment décrits dans les brevets français 2.252.840 et 2.368.508;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8: 1 et 1,4: 1; le polyaminoamide en résultant étant amené à réagir avec l'épichlorhydrine dans un rapport molaire d'épichlorhydrine par rapport au groupement amine secondaire du polyaminoamide compris entre 0,5: 1 et 1,8: 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylène-triamine.
(9) les cyclohomopolymères de méthyl diallyl amine ou de diméthyl diallylammonium tels que les homopolymères comportant comme constituant principal de la chaîne des motifs répondant aux formules (VI) ou (VI'): 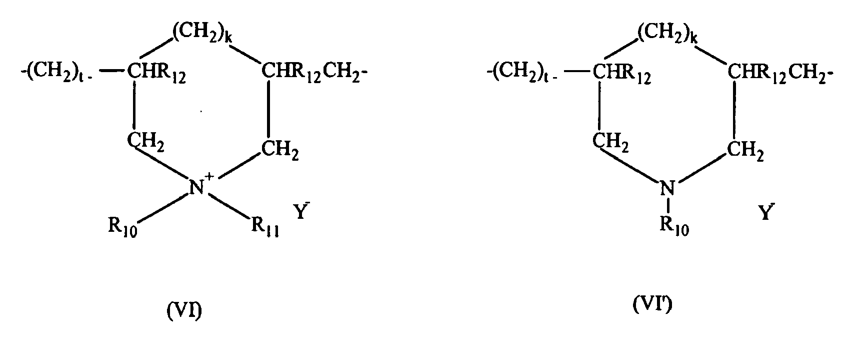 formules dans lesquelles k et t sont égaux à 0 ou 1, la somme k + t étant égale à 1; R₁₂ désigne un atome d'hydrogène ou un radical méthyle; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle; Y^{Θ} est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères défnis ci-dessus, on peut citer plus particulièrement l'homopolymère de chlorure de diméthyldiallylammonium vendu sous la dénomination "Merquat 100" par la société Merck.
(10) le polymère de diammonium quaternaire contenant des motifs récurants répondant à la formule: 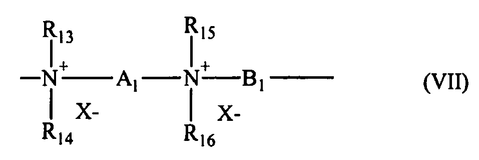 formule (VII) dans laquelle:
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire; A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et XΘ désigne un anion dérivé d'un acide minéral ou organique; A₁, R₁₃ et R₁₅ peuvent former avec les deux atomes d'azote auxquels ils sont rattachés un cycle pipérazinique, en outre si A₁ désigne un radical alkylène ou hydroxyalkylène linéaire ou ramifié, saturé ou insaturé, B₁ peut également désigner un groupement (CH₂)ₙ-CO-D-OC-(CH₂)ₙ dans lequel D désigne:
      a) un reste de glycol de formule: -O-Z-O-, où Z désigne un radical hydrocarboné linéaire ou ramifié ou un groupement répondant à l'une des formules suivantes:

         -(CH₂-CH₂-O)ₓ-CH₂-CH₂-

         -[CH₂-CH(CH₃)-O]_{y}-CH₂-CH(CH₃)-

         où x et y désignent un nombre entier de 1 à 4, représentant un degré de polymérisation défini et unique ou un nombre quelconque de 1 à 4 représentant un degré de polymérisation moyen;
      b) un reste de diamine bis-secondaire tel qu'un dérivé de pipérazine;
      c) un reste de diamine bis-primaire de formule: -NH-Y-NH-, où Y désigne un radical hydrocarboné linéaire ou ramifié, ou bien le radical bivalent -CH₂-CH₂-S-S-CH₂-CH₂-;
      d) un groupement uréylène de formule: -NH-CO-NH-; De préférence, XΘ est un anion tel que le chlorure ou le bromure.
      Ces polymères ont une masse moléculaire en nombre généralement comprise entre 1000 et 100000.
      Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) les polymères de polyammonium quaternaires constitués de motifs de formule (VIII): 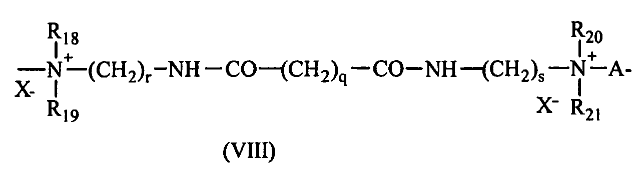 formule dans laquelle:
   R₁₈, R₁₉, R₂₀ et R₂₁, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un radical d'un dihalogénure ou représente de préférence -CH₂CH₂-O-CH₂-CH₂-.
   De tels composés sont notamment décrits dans la demande de brevet EP-A-122 324.
   On peut par exemple citer parmi ceux-ci, les produits "Mirapol A 15", "Mirapol AD1", "Mirapol AZ1" et "Mirapol 175" vendus par la société Miranol.
(12) les homopolymères ou copolymères dérivés des acides acrylique ou méthacrylique et comportant des motifs: 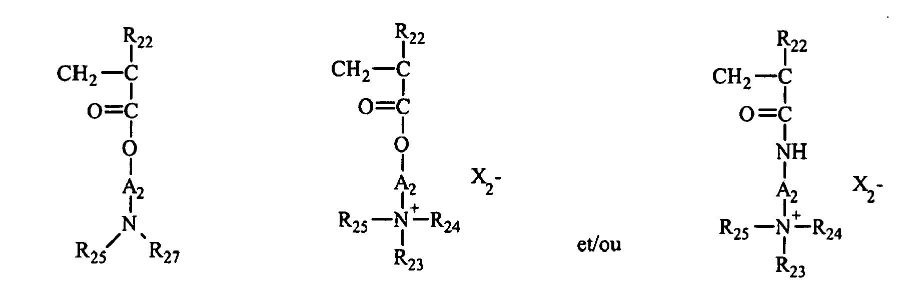 dans lesquels les groupements R₂₂ désignent indépendamment H ou CH₃, les groupements A₁ désignent indépendamment un groupe alcoyle linéaire ou ramifié de 1 à 6 atomes de carbone ou un groupe hydroxyalcoyle de 1 à 4 atomes de carbone,
   les groupements R₂₃, R₂₄, R₂₅, identiques ou différents, désignant indépendamment un groupe alcoyle de 1 à 18 atomes de carbone ou un radical benzyle,
   les groupements R₂₆ et R₂₇ représentent un atome d'hydrogène ou un groupement alcoyle de 1 à 6 atomes de carbone,
   X₂Θ désigne un anion, par exemple méthosulfate ou halogénure, tel que chlorure ou bromure.
   Le ou les comonomères utilisables dans la préparation des copolymères correspondants appartiennent à la famille des acrylamides, méthacrylamides, diacétone acrylamides, acrylamides et méthacrylamides substitués à l'azote par des alcoyles inférieurs, des esters d'alcoyles, des acides acrylique ou méthacrylique, la vinylpyrrolidone ou des esters vinyliques.
(13) Les polymères quaternaires de vinylpyrrolidone et de vinylimidazole tels que par exemple les produits commercialisés sous les dénominations Luviquat FC 905, FC 550 et FC 370 par la société B.A.S.F..
(14) Les polyamines comme le Polyquart H vendu par HENKEL, référencé sous le nom de « POLYETHYLENEGLYCOL (15) TALLOW POLYAMINE » dans le dictionnaire CTFA.
(15) Les polymères réticulés de chlorure de méthacryloyloxyéthyltriméthylammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE SC 92» par la Société ALLIED COLLOIDS. On peut également utiliser un homopolymère réticulé du chlorure de méthacryloyloxyéthyl triméthylammonium contenant environ 50% en poids de l'homopolymère dans de l'huile minérale. Cette dispersion est commercialisée sous le nom de « SALCARE SC 95 » par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Selon l'invention, on peut utiliser plus particulièrement les polymères choisis parmi le Mirapol, le composé de formule (VII) dans laquelle R₁₃, R₁₄, R₁₅ et R₁₆ représentent le radical méthyle, A₁ représente le radical de formule -(CH₂)₃- et B₁ représente le radical de formule -(CH₂)₆- et XΘ représente l'anion chlorure (nommé ultérieurement Mexomère PO) et le composé de formule (VII) dans laquelle R₁₃ et R₁₄ représentent le radical éthyle, R₁₅ et R₁₆ représentent le radical méthyle, A₁ et B₁ représentent le radical de formule -(CH₂)₃₋et X^{Θ} représente l'anion bromure (nommé ultérieurement Mexomère PAK).

Parmi tous les polymères cationiques susceptibles d'être utilisés, on préfère mettre en oeuvre les composés décrits précédemment aux points (3) et (4).

Selon l'invention, le ou les polymères cationiques peuvent représenter de 0,001% à 10% en poids, de préférence de 0,005% à 5% en poids, et encore plus préférentiellement de 0,01% à 3% en poids, du poids total de la composition finale.

Le milieu aqueux peut contenir, outre de l'eau, des solvants cosmétiquement acceptables, différents de l'agent propénétrant tels que des monoalcools, des polyalcools, des éthers de glycol utilisés seuls ou en mélange.

Parmi ces solvants, on peut plus particulièrement mentionner le polyéthylène glycol, le glycérol et le sorbitol. Les solvants sont utilisés de préférence dans des proportions de 0,5 à 10% en poids par rapport au poids total de la composition.

Le pH des compositions est de préférence compris entre 2 et 9 et en particulier entre 3 et 8. Pour le propionate de clobetasol, le pH est avantageusement compris entre 5,5 et 6,5. Il est ajusté par des agents alcalinisants ou acidifiants cosmétiquement acceptables.

Les compositions conformes à l'invention peuvent en outre contenir d'autres adjuvants utilisés dans des compositions moussantes telles que des shampooings, et notamment des céramides, telles que celles décrites dans le brevet français FR 2 673 179, des glycocéramides, des agents tensioactifs non-ioniques bien connus qui peuvent être choisis parmi les alcools, les alphadiols, les alkylphénols, les acides gras polyéthoxylés, polypropoxylés ou polyglycérolés à chaîne grasse comportant par exemple 8 à 18 atomes de carbone, le nombre de groupements d'oxyde d'éthylène et d'oxyde de propylène pouvant aller en particulier de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras; les amides gras polyéthoxylés ayant de préférence 2 à 30 moles d'oxyde d'éthylène, les amides gras polyglycérolés comportant en moyenne 1 à 5 groupements glycérol et en particulier 1,5 à 4 groupements glycérol; les amines grasses polyéthoxylées ayant de préférence 2 à 3 moles d'oxyde d'éthylène; les esters d'acides gras du sorbitan oxyéthylénés ayant de 2 à 30 moles d'oxyde d'éthylène; les esters d'acides gras du sucrose, les esters d'acides gras du polyéthylèneglycol, les alkylpolyglycosides, les dérivés de N-alkyl glycamine, les oxydes d'amines tels que les oxydes d'alkyl(C₁₀-C₁₄) amines ou les oxydes de N-acylaminopropylmorpholine. Les alkylpolyglycosides et les alcools alfadiols alkylphénols d'acides gras polyglycérolés sont plus particulièrement préférés.

Les compositions peuvent également contenir des agents épaisissants choisis notamment parmi l'alginate de sodium, la gomme arabique, les dérivés cellulosiques tels que la méthylcellulose, l'hydroxyméthylcellulose, l'hydroxyéthylcellulose, l'hydroxypropylcellulose, l'hydroxypropylméthylcellulose, la gomme de guar ou ses dérivés, les gommes de xanthane, les scléroglucanes, les acides polyacryliques réticulés, les polyuréthanes, les copolymères à base d'acide ou d'anhydride maléique, les épaississants associatifs porteurs de chaînes grasses de type naturel comme le produit commercialisé sous la dénomination NATRASOL PLUS ou synthétiques comme les produits commercialisés sous la dénomination PEMULEN.

L'épaississant peut également être obtenu par mélange du polyéthylèneglycol et de stéarates ou de distéarates de polyéthylène glycol ou par mélange d'esters phosphoriques et d'amides.

Les compositions conformes à l'invention peuvent également contenir des colorants, des agents modificateurs de viscosité, des agents nacrants, des agents hydratants, des agents antipelliculaires, des agents antiséborrhéiques, des filtres solaires, des protéines, des vitamines, des α-hydroxyacides, des sels, des parfums, des agents conservateurs, des séquestrants, des adoucissants, des modificateurs de mousse, des détoxifiants ou leurs mélanges.

Des agents conditionneurs peuvent également être utilisés tels que les huiles naturelles hydrogénées ou non, synthétiques ou non, hydrocarbonées, cycliques ou aliphatiques, linéaires ou ramifiées (saturées ou non), les silicones volatiles ou non, organomodifiées ou non, solubles ou non, des huiles perfluorées ou fluorées, les polybutènes et polyisobutènes, des esters gras se présentant sous forme liquide, pâteuse ou solide, les esters d'alcools polyhydriques, des glycérides, les cires naturelles ou synthétiques, des gommes et résines de silicones, les sels d'ammonium quaternaire tels que par exemple le composé classé dans le dictionnaire CTFA, 7ème édition, 1998, sous la dénomination Quaternium-22 et commercialisé sous la dénomination "Ceraphyl 60", ou le mélange de ces différents agents.

Dans le cadre de la présente invention, les compositions sont plus particulièrement sous la forme de liquides, éventuellement épaissis.

Elles peuvent être utilisées en l'état ou être diluées avant utilisation.

Les compositions conformes à l'invention sont plus particulièrement utilisées comme shampooings pour le traitement des cheveux ou du cuir chevelu.

On applique, dans ce cas, préférentiellement la composition sur les cheveux mouillés ou secs puis on procède à un léger massage au cours duquel se forme une mousse, puis on rince et, éventuellement, on applique une nouvelle fois le shampooing suivi d'un nouveau rinçage à l'eau.

Un objet de l'invention est aussi une composition moussante telle que définie ci-dessus pour son application comme médicament.

La présente invention a aussi pour objet l'utilisation d'une composition telle que définie ci-dessus pour la fabrication d'un médicament destiné à traiter les affections du cuir chevelu.

Les compositions selon l'invention sont tout particulièrement indiquées dans le traitement des éczémas, des érythrodermies eczémateuses ou psoriasiques, des lésions prurigineuses, du lupus érythémateux chronique, du psoriasis et du parapsoriasis en plaques.

Ces traitements nécessitent généralement une application telle que décrite ci-dessus 2 à 3 fois par semaine.

On va maintenant donner à titre d'illustration et sans aucun caractère limitatif plusieurs exemples de compositions selon l'invention.

### EXEMPLE II

On prépare le shampooing suivant:
- Dehyton AB 30 (cocoylbétaïne à 32% M.A.) 6 g
- Jaguar C162®(hydroxyméthylguar
   trimethylammonium) 0,5 g
- Sipon A0S 225 UP® (lauryléther sulfate
   de sodium à 28% M.A.) 43 g
- Ethanol (95/96%) 10 g
- Propionate de clobétasol 0,05 g
- Chlorure de benzalkonium 0,005 g
- Acide lactique qs pH6
- Eau déminéralisée qs 100 g

### EXEMPLE IV

On prépare le shampooing suivant:
- Sipon AOS 225 UP® (lauryléther sulfate de
   sodium à 28% M.A.) 43 g
- Dehyton AB 30® (cocoylbetaïne à 31% M.A.) 6 g
- Ethanol (95/96%) 10 g
- Chlorure de benzalkonium 0,01 g
- Jaguar C 162® (hydroxymethylguar
   trimethylammonium) 0,5 g
- Propionate de clobétasol 0,05 g
- Acide lactique qs pH 6
- eau déminéralisée qs 100 g

### EXEMPLE V

On prépare le shampooing suivant:
- Celquat SC 240® (Polyquaternium 10) 2 g
- Texapon N70® (Lauryléther sulfate
   de sodium (2 moles OE) à 70% M.A.) 17 g
- Dehyton AB 30® (Cocoylbétaïne à 32% M.A.) 6 g
- Rewoquat B50® (Chlorure de
   benzalkonium 50%) 0,01 g
- Acide citrique, 1H₂0 0,24 g
- Citrate de sodium, 2H₂0 2,6 g
- Ethanol (95/96%) 10 g
- Propionate de clobétasol 0,05 g
- Eau déminéralisée qs 100 g

### EXEMPLE VI

On prépare le shampooing suivant:
- Celquat SC 240® (Polyquaternium 10) 2 g
- Texapon N70® (Lauryléther sulfate
   de sodium (2 moles OE) à 70% M.A.) 17 g
- Dehyton AB 30® (Cocoylbétaïne à 32% M.A.) 6 g
- Rewoquat B50® (Chlorure de
   benzalkonium 50%) 0,01 g
- Ethanol (95/96%) 10 g
- Propionate de clobétasol 0,05 g
- Eau déminéralisée qsp 100 g

### EXEMPLE VII

On prépare le shampooing suivant:
- Acide citrique, 1H₂0 0,24 g
- Citrate de sodium, 2H₂ 2,6 g
- Parahydroxybenzoate de méthyl 0,1 g
- Celquat SC 240® (polyquaternium 10) 2 g
- Texapon N70® (Lauryléther sulfate
   de sodium (2 moles OE) à 70% M.A.) 17 g
- Dehyton AB 30® (Cocoylbétaïne à 32% M.A.) 6 g
- Propionate de clobétasol 0,05 g
- Ethanol (95/96%) 10 g
- Eau purifiée qsp 100 g

### EXEMPLE VIII

On prépare le shampooing suivant:
- Acide citrique 0,24 g
- Citrate de sodium 2,6 g
- Celquat SC 240® (Polyquaternium 10) 2 g
- Texapon N70®(Lauryléther sulfate
   de sodium (2 moles OE) à 70% M.A.) 17 g
- Dehyton AB 30® (Cocoylbétaïne à 32% M.A.) 6 g
- Propionate de Clobetasol 0,05 g
- Ethanol (95/96%) 10 g
- Eau purifiée qsp 100 g

### EXEMPLE IX

On prépare le shampooing suivant:
- Jaquar C 162®(hydroxymethylguar
   trimethylammonium) 0,5 g
- Chimexane HC (Cocoylbétaïne
   à 32% M.A.) 6,0 g
- Sipon AOS 225 UP® (lauryléther sulfate de
   sodium à 28% M.A.) 43,0 g
- Ethanol (95/96%) 10,0 g
- Propionate de clobétasol 0,05 g
- Rewoquat B50 (Chlorure de
   benzalkonium 50%) 0,01 g
- Eau purifiée qsp 100. g

### EXEMPLE XIV

On prépare le shampooing suivant:
- Texapon N70® (Lauryléther sulfate
   de sodium (2 moles OE) à 70% M.A.) 17 g
- Dehyton AB 30® (Cocoylbétaïne à 32% M.A.) 6 g
- Methocel E4M(hydroxy propyl
   methyl cellulose) 1 g
- Acide citrique, 1H₂0 0,24 g
- Citrate de sodium, 2H₂ 0 2,6 g
- Ceraphyl 60 (Quaternium 22) 0,5 g
- Propionate de clobétasol 0,05 g
- Ethanol (95/96%) 10 g
- Eau déminéralisée qs 100 g

Les compositions des exemples II à XIV ci-dessus sont stables au stockage et présentent un effet moussant satisfaisant.

Une étude clinique, dans laquelle une composition selon l'exemple IX a été utilisée comme shampooing une fois par jour pendant deux semaines, le shampooing étant appliqué sur cheveux mouillés et laissé en contact pendant 10 minutes, pour être ensuite rincé, a permis de constater chez des patients atteints de psoriasis, une réduction au niveau du cuir chevelu des érythèmes de 37%, de la desquamation de 47%, des hyperkératoses de 50% et des prurits de 57%.

## Revendications

1. Composition moussante pour le lavage et le traitement des cheveux et/ou du cuir chevelu, **caractérisée par le fait qu'**elle contient dans un milieu aqueux:
- du propionate de clobétasol,
- au moins un tensioactif anionique choisi parmi le lauryléthersulfate de sodium et le laurylsulfate de sodium,
- au moins un tensioactif amphotère choisi parmi les cocobétaïnes, et
- l'éthanol,
ladite composition présentant un pH compris entre 5,5 et 6,5.

2. Composition selon la revendication 1, **caractérisée par le fait que** le propionate de clobétasol est le 17 propionate de clobétasol.

3. Composition selon la revendication 1 ou 2, **caractérisée par le fait que** le propionate de clobétasol est utilisé dans des proportions de 0,001 à 5%, préférentiellement entre 0,01 à 0,3% et plus préférentiellement entre 0,05 et 0,1% en poids par rapport au poids total de la composition.

4. Composition selon l'une quelconque des revendications 1 à 3, **caractérisée par le fait que** le tensioactif anionique est utilisé dans des proportions de 0,05 et 50%, de préférence entre 1 et 30% et plus préférentiellement entre 2 et 25% de M.A. en poids par rapport au poids total de la composition.

5. Composition selon l'une quelconque des revendications 1 à 4, **caractérisée par le fait que** le tensioactif amphotère est utilisé dans des proportions de 0,01 et 30%, de préférence entre 0,5 et 20% et plus préférentiellement entre 1 et 15% en M.A. en poids par rapport au poids total de la composition.

6. Composition selon l'une quelconque des revendications 1 à 5, **caractérisée par le fait que** l'éthanol est utilisé dans des concentrations comprises entre 0,1 et 25% et préférentiellement comprises entre 5 et 10% en poids par rapport au poids total de la composition.

7. Composition selon l'une quelconque des revendications 1 à 6, **caractérisée en ce que** le ratio entre les proportions en M.A. des tensioactifs anioniques et amphotères est compris entre 1 et 20 et, préférentiellement entre 2 et 10.

8. Composition selon l'une quelconque des revendications 1 à 7, **caractérisée en ce que** le ratio entre la proportion en M.A. des tensioactifs anioniques et la proportion d'éthanol est compris entre 0,1 et 10, préférentiellement entre 0,5 et 5.

9. Composition selon l'une quelconque des revendications 1 à 8, **caractérisée par le fait que** la composition contient en outre des polymères cationiques choisis parmi les dérivés d'éther de celluloses cationiques et les polysaccharides cationiques.

10. Composition selon la revendication 9, **caractérisée par le fait que** le polymère cationique représente 0,001 % à 10 % en poids, de préférence de 0,005 % à 5 % en poids, et encore plus préférentiellement de 0,01 % à 3' % en poids, par rapport au poids total de la composition finale.

11. Composition selon l'une quelconque des revendications 1 à 10, **caractérisée par le fait qu'**elle contient également du quaternium-22.

12. Composition selon l'une quelconque des revendications 9 à 11, **caractérisée par le fait qu'**elle consiste en :
- 0,05 g. de propionate de clobétasol,
- 11,9 g. de lauryléther sulfate de sodium (2 moles OE),
- 1,92 g. de cocoylbétaïne,
- 9,6 g. d'éthanol,
- 2 g. de polyquaternium-10
- 0,24 g. d'acide citrique
- 2,6 g. de citrate de sodium,
- le complément jusqu'à 100 g. étant de l'eau:

13. Composition selon l'une quelconque des revendications 1 à 12, pour son application comme médicament.

14. Utilisation de la composition telle que définie dans l'une quelconque des revendications 1 à 13, pour la fabrication d'un médicament destiné à traiter les affections du cuir chevelu.

## Claims

1. Foaming composition for washing and treating the hair and/or scalp, **characterized in that** it comprises, in an aqueous medium:
- clobetasol propionate,
- at least one anionic surfactant chosen from sodium lauryl ether sulphate and sodium lauryl sulphate,
- at least one amphoteric surfactant chosen from coco betaines, and
- ethanol,
the said composition exhibiting a pH of between 5.5 and 6.5.

2. Composition according to Claim 1, **characterized in that** the clobetasol propionate is clobetasol 17-propionate.

3. Composition according to Claim 1 or 2, **characterized in that** the clobetasol propionate is used in proportions of 0.001 to 5% by weight, preferably between 0.01 and 0.3% by weight and most preferably between 0.05 and 0.1% by weight, with respect to the total weight of the composition.

4. Composition according to any one of Claims 1 to 3, **characterized in that** the anionic surfactant is used in proportions of 0.05 to 50% of A.M. by weight, preferably between 1 and 30% of A.M. by weight and more preferably between 2 and 25% of A.M. by weight, with respect to the total weight of the composition.

5. Composition according to any one of Claims 1 to 4, **characterized in that** the amphoteric surfactant is used in proportions of 0.01 and 30% of A.M. by weight, preferably between 0.5 and 20% of A.M. by weight and more preferably between 1 and 15% of A.M. by weight, with respect to the total weight of the composition.

6. Composition according to any one of Claims 1 to 5, **characterized in that** the ethanol is used in concentrations of between 0.1 and 25% by weight and preferably of between 5 and 10% by weight, with respect to the total weight of the composition.

7. Composition according to any one of Claims 1 to 6, **characterized in that** the ratio between the proportions as A.M. of the anionic and amphoteric surfactants is between 1 and 20 and preferably between 2 and 10.

8. Composition according to any one of Claims 1 to 7, **characterized in that** the ratio of the proportion as A.M. of the anionic surfactants to the proportion of ethanol is between 0.1 and 10, preferably between 0.5 and 5.

9. Composition according to any one of Claims 1 to 8, **characterized in that** the composition additionally comprises cationic polymers chosen from cationic cellulose ether derivatives and cationic polysaccharides.

10. Composition according to Claim 9, **characterized in that** the cationic polymer represents from 0.001% to 10% by weight, preferably from 0.005% to 5% by weight and more preferably still from 0.01% to 3% by weight, with respect to the total weight of the final composition.

11. Composition according to any one of Claims 1 to 10, **characterized in that** it also comprises quaternium-22.

12. Composition according to any one of Claims 9 to 11, **characterized in that** it is composed of:
- 0.05 g of clobetasol propionate,
- 11.9 g of sodium lauryl ether sulphate (2 mol EO),
- 1.92 g of cocoyl betaine,
- 9.6 g of ethanol,
- 2 g of polyquaternium-10,
- 0.24 g of citric acid,
- 2.6 g of sodium citrate,
- the remainder up to 100 g being water.

13. Composition according to any one of Claims 1 to 12 for its application as medicament.

14. Use of the composition as defined in any one of Claims 1 to 13 in the manufacture of a medicament intended to treat conditions of the scalp.

## Patentansprüche

1. Schaumbildende Zusammensetzung für die Reinigung und Behandlung der Haare und/oder der Kopfhaut, **dadurch gekennzeichnet, dass** sie ein einem wässrigen Medium enthält:
- Clobetasolpropionat,
- mindestens einen anionischen grenzflächenaktiven Stoff, der unter Natriumlaurylethersulfat und Natriumlaurylsulfat ausgewählt ist,
- mindestens einen amphoteren grenzflächenaktiven Stoff, der unter den Cocobetainen ausgewählt ist, und
- Ethanol,
wobei die Zusammensetzung einen pH-Wert von 5,5 bis 6,5 aufweist.

2. Zusammensetzung nach Anspruch 1, **dadurch gekennzeichnet, dass** das Clobetasolpropionat das Clobetasol-17-propionat ist.

3. Zusammensetzung nach Anspruch 1 oder 2, **dadurch gekennzeichnet, dass** das Clobetasolpropionat in Mengenanteilen von 0,001 bis 5 Gew.-%, vorzugsweise 0,01 bis 0,3 Gew.-% und besonders bevorzugt 0,05 bis 0,1 Gew.-%, bezogen auf das Gesamtgewicht der Zusammensetzung, verwendet wird.

4. Zusammensetzung nach einem der Ansprüche 1 bis 3, **dadurch gekennzeichnet, dass** der anionische grenzflächenaktive Stoff in Mengenanteilen der wirksamen Substanz (wS) von 0,05 bis 50 Gew.-%, vorzugsweise 1 bis 30 Gew.-% und besonders bevorzugt 2 bis 25 Gew.-%, bezogen auf das Gesamtgewicht der Zusammensetzung, verwendet wird.

5. Zusammensetzung nach einem der Ansprüche 1 bis 4, **dadurch gekennzeichnet, dass** der amphotere grenzflächenaktive Stoff in Mengenanteilen wS von 0,01 bis 30 Gew.-%, vorzugsweise 0,5 bis 20 Gew.-% und besonders bevorzugt 1 bis 15 Gew.-%, bezogen auf das Gesamtgewicht der Zusammensetzung, verwendet wird.

6. Zusammensetzung nach einem der Ansprüche 1 bis 5, **dadurch gekennzeichnet, dass** das Ethanol in Konzentrationen im Bereich von 0,1 bis 25 Gew.-% und vorzugsweise 5 bis 10 Gew.-%, bezogen auf das Gesamtgewicht der Zusammensetzung, verwendet wird.

7. Zusammensetzung nach einem der Ansprüche 1 bis 6, **dadurch gekennzeichnet, dass** das Verhältnis der als wS ausgedrückten Mengenanteile der anionischen und amphoteren grenzflächenaktiven Stoffe im Bereich von 1 bis 20 und vorzugsweise 2 bis 10 liegt.

8. Zusammensetzung nach einem der Ansprüche 1 bis 7, **dadurch gekennzeichnet, dass** das Verhältnis des als wS ausgedrückten Mengenanteils der anionischen grenzflächenaktiven Stoffe und des Mengenanteils des Ethanols im Bereich von 0,1 bis 10 und vorzugsweise 0,5 bis 5 liegt.

9. Zusammensetzung nach einem der Ansprüche 1 bis 8, **dadurch gekennzeichnet, dass** die Zusammensetzung ferner kationische Polymere enthält, die unter kationischen Celluloseetherderivaten und kationischen Polysacchariden ausgewählt ist.

10. Zusammensetzung nach Anspruch 9, **dadurch gekennzeichnet, dass** das kationische Polymer 0,001 bis 10 Gew.-%, vorzugsweise 0,005 bis 5 Gew.-% und noch bevorzugter 0,01 bis 3 Gew.-%, bezogen auf das Gesamtgewicht der Zusammensetzung, ausmacht.

11. Zusammensetzung nach einem der Ansprüche 1 bis 10, **dadurch gekennzeichnet, dass** sie ferner Quaternium-22 enthält.

12. Zusammensetzung nach einem der Ansprüche 9 bis 11, **dadurch gekennzeichnet, dass** sie besteht aus:
- 0,05 g Clobetasolpropionat,
- 11,9 g Natriumlaurylethersulfat (2 mol EO),
- 1,92 g Cocoylbetain,
- 9,6 g Ethanol,
- 2 g Polyquaternium-10,
- 0,24 g Citronensäure,
- 2,6 g Natriumcitrat,
- wobei der auf 100 g fehlende Rest Wasser ist.

13. Zusammensetzung nach einem der Ansprüche 1 bis 12 zur Verwendung als Arzneimittel.

14. Verwendung der Zusammensetzung nach einem der Ansprüche 1 bis 13 für die Herstellung eines Arzneimittels für die Behandlung der Erkrankungen der Kopfhaut.
